# EUROPEAN PATENT APPLICATION

(11) **EP 1 870 095 A1**
(43) Date of publication of application: **26.12.2007**
(21) Application number: 06730264.6
(22) Date of filing: 28.03.2006
(51) Int. Cl.: A61K 31/09, A61K 31/122, A61P 9/10, A61P 39/06

(54) **COMPOSITION FOR INCREASING ANTI-OXIDATION ACTIVITY IN BLOOD**

(30) Priority: 29.03.2005 JP 2005094927
(71) Applicant: Kaneka Corporation, Kita-ku Osaka-shi, Osaka 530-8288 (JP)
(72) Inventor: KUBO, Hiroshi, ogo, 6550048 (JP); FUJII, Kenji, Hyogo, 6511202 (JP); HOSOE, Kazunori, 025 (JP)
(74) Representative: Hart Davis, Jason
(86) International application number: PCT/JP2006/306316
(87) International publication number: WO 2006/104153

(57) **Abstract**

The present invention provides a composition capable of maintaining antioxidative activity in blood high by keeping the reduced coenzyme Q₁₀ ratio in blood high after taking coenzyme Q₁₀ even in aged mammals.

The present invention relates to a composition which comprises reduced coenzyme Q₁₀ as an active component and enhances the antioxidative activity in blood of aged mammals.

## Description

### TECHNICAL FIELD

The present invention relates to a composition which comprises reduced coenzyme Q₁₀ as an active component and enhances the antioxidative activity in blood of aged mammals.

### BACKGROUND ART

Active oxygen contributes to physiologically important metabolism and plays important roles in vital activities on some occasions by, for example, producing biologically active components, killing pathogenic bacteria, and playing an important role involving anticancer action. On the other hand, excess generation of active oxygen, which cannot be controlled, is considered to cause some damage to the living body via denaturation of nucleic acids and/or proteins, or a peroxidation reaction of lipids. The condition wherein the amount of active oxygen is not controlled and excess amount of active oxygen occurs is called oxidative stress condition. In recent years, it has become clear that oxidative stress causes some adverse effect on various diseases.

As the diseases related to oxidative stress, there has been reported arteriosclerosis, cancer, cerebral ischemia, hepatic dysfunction, diabetes mellitus, neurological disorder, renal disorder, hepatic cirrhosis, arthritis, retinopathy of prematurity, ocular uveitis, retinal siderosis, senile cataract, dysfunction due to an adverse effect derived from radiation therapy, asbestosis, bronchial injury due to smoking, dysfunction due to an adverse effect derived from carcinostatic drug use, cerebral edema, pulmonary edema, pedal edema, cerebral infarction, hemolytic anemia, progeria, epilepsy, Alzheimer's disease, Down's syndrome, Parkinson's disease, Behcet's disease, Crohn's disease, Kawasaki disease, Weber-Christian disease, collagen disease, progressive systemic sclerosis, dermatitis herpetiformis, immunologic deficiency syndrome, and the like diseases. To such diseases, availability of antioxidants (radical scavengers) which are considered to have oxidative stress-reducing effect has been examined for years, and anti-inflammatory drugs showing a radical scavenging effect have been developed. In addition, according to the fact that a novel substance (Radicut) showing an antioxidative effect was recently certified as a pharmaceutical preparation, availability of the antioxidants in reducing oxidative stress can be said obvious.

Coenzyme Q₁₀ is a component widely distributed in living organisms, and performs important function as a component of the electron transport system of mitochondria, which is one of organelle. Also, coenzyme Q₁₀ was found to have another important effect, namely the antioxidative effect, and has drawn attention. Having the antioxidative activity, coenzyme Q₁₀ is expected to have prevention and improvement effects on the above-mentioned diseases which are supposedly related to oxidative stress, and has been increasingly taken as a supplement in recent years.

Coenzyme Q₁₀ occurs as its oxidized form or reduced form. One showing the antioxidative activity is reduced coenzyme Q₁₀ and therefore no antioxidative activity is exerted even when there are enormous amount of oxidized forms (Non-Patent Document 1 and 2). Accordingly, at the time of oxidized coenzyme Q₁₀ intake, oxidized coenzyme Q₁₀ is necessary to be converted into its reduced form in the living body for showing its antioxidative activity. That is, for enhancing the antioxidative activity and reducing oxidative stress in the living body by taking coenzyme Q₁₀, it is very important that coenzyme Q₁₀ occurs as its reduced form in the organs and blood of the living body, where coenzyme Q₁₀ is transported via blood.

Generally, it is said that oxidized coenzyme Q₁₀ orally taken as a supplement is absorbed into the small intestine, enzymatically reduced to reduced coenzyme Q₁₀ with consuming reducing equivalent of reduced nicotineamide adenine dinucleotide phosphate (NADPH) and the like as a substrate, and then released into blood in the form integrated in lipoproteins (Non-Patent Document 1 and 3). That is, oxidized coenzyme Q₁₀ intake also increases reduced coenzyme Q₁₀ in the living body. On the other hand, an oxidized form and reduced form of coenzyme Q₁₀ coexist in plasma. In recent years, it has become clear that the reduced coenzyme Q₁₀ ratio in plasma is useful as an oxidative stress marker. This is attributed to the fact that reduced coenzyme Q₁₀ which has been once oxidized is not reduced again in plasma since no enzyme capable of reducing oxidized coenzyme Q₁₀ exists in blood. It has been reported that, as for liver diseases known to be exacerbated due to oxidative stress, the oxidized coenzyme Q₁₀ ratio increases depending on the severity of the disease (Non-Patent Document 4). In this manner, the effect of oxidative stress exposure is shown in the reduced coenzyme Q₁₀ ratio in plasma, and thus it is supposed that increased ratio of reduced coenzyme Q₁₀ in plasma results from an enhanced antioxidative activity in the living body.

Non-Patent Document 5 discloses that, when a 35-year-old healthy person took oxidized coenzyme Q₁₀, the ratio of reduced coenzyme Q₁₀ to oxidized coenzyme Q₁₀ was unchanged despite the amount of reduced coenzyme Q₁₀ in plasma increased. That is, although oxidized coenzyme Q₁₀ is reduced and converted into its reduced form during adsorption, the antioxidative activity in the living body cannot be enhanced and the ratio of reduced coenzyme Q₁₀ to the total amount of coenzymes in plasma cannot increase. No composition showing such activity has been known.

Furthermore, for aged humans and aged mammals considered to be exposed to oxidative stress for years and have lowered antioxidative activity, no method nor composition for increasing the reduced coenzyme Q₁₀ ratio to the total amount of coenzymes in plasma has also been unknown.
Non-Patent Document 1: "Saibo kara genki ni naru (Revive cells)" Coenzyme Q10 General guide book 39-41, page 48 (2003)
Non-Patent Document 2:Vitamin, vol.75, No.5 and 6, page 279 (2001)
Non-Patent Document 3: Steven Greenberg, et al, J. Clin. Pharmacol., 30 596-608 (1990)
Non-Patent Document 4: Yorihiro Yamamoto, et al, BioFactors, 9, 241-246 (1999)
Non-Patent Document 5: Detlef Mohr, et al. Biochimica et Biophysica Acta, 1126, 247-254 (1992)

### SUMMARY OF THE INVENTION

Accordingly, the present invention has for its object to provide a composition capable of maintaining antioxidative activity in blood high, even in aged mammals, by keeping the reduced coenzyme Q₁₀ ratio in blood high after coenzyme Q₁₀ intake.

The present inventors have made intensive investigations to solve the above-mentioned subjects and, as a result, they surprisingly found that the reduced coenzyme Q₁₀ ratio in blood can be kept high even in aged mammals by the intake of a composition comprising reduced coenzyme Q₁₀. Such and other findings have led to completion of the present invention.

Thus, the present invention relates to
a composition
which comprises reduced coenzyme Q₁₀ represented by the following formula (1), as an active component.

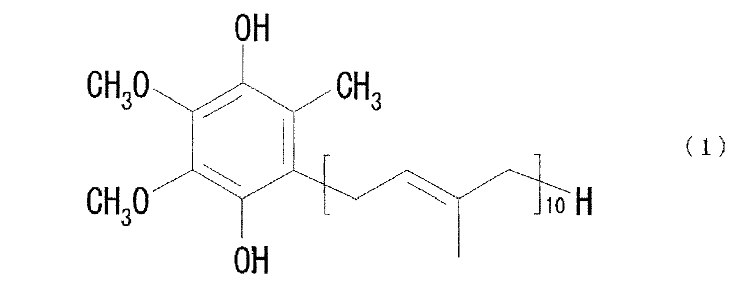

Also, the present invention relates to
a method for producing a composition
which comprises mixing reduced coenzyme Q₁₀ with a carrier acceptable as a pharmaceutical preparation or food.

The above-mentioned composition is preferable for administration to aged mammals, and is also preferable for enhancing the antioxidative activity in blood of aged mammals.

Furthermore, the present invention relates to
a method for enhancing the antioxidative activity in blood
which comprises administering reduced coenzyme Q₁₀.

The above method is preferably used for aged mammals.

### DETAILED DESCRIPTION OF THE INVENTION

The present invention is now described in detail.

The composition of the invention which comprises reduced coenzyme Q₁₀ as an active component and enhances the antioxidative activity in blood of aged mammals is described.

Reduced coenzyme Q₁₀ according to the invention is represented by the above formula (1).

The method for obtaining reduced coenzyme Q₁₀ is not particularly restricted and employable as such methods are, for example, a method comprising obtaining coenzyme Q₁₀ in the conventional manner such as synthesis, fermentation or extraction from natural sources, and then concentrating a reduced coenzyme Q₁₀ fraction in the effluent by chromatography. Also employable for obtaining reduced coenzyme Q₁₀ is a method comprising reacting an existing highly pure oxidized coenzyme Q₁₀ with an ordinary reducing agent such as sodium borohydride or sodium dithionite (sodium hydrosulfite). Further, a strain containing reduced coenzyme Q₁₀, and the like can also be used.

In the invention, the composition which comprises reduced coenzyme Q₁₀ as an active component may further comprise oxidized coenzyme Q₁₀ represented by the following formula (2). 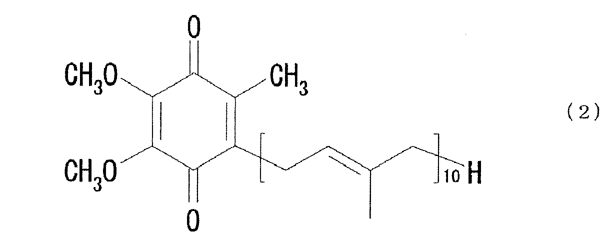 In this case, the content of reduced coenzyme Q₁₀ to the total coenzyme Q₁₀ is not particularly restricted, but the lower limit thereof is preferably not lower than 80% by weight, more preferably not lower than 90% by weight, still more preferably not lower than 95% by weight, and particularly preferably not lower than 98% by weight. The upper limit of reduced coenzyme Q₁₀ is preferably not higher than 100% by weight.

The ratio of the reduced form to the total coenzyme Q₁₀ is generally determined by a method comprising quantifying oxidized coenzyme Q₁₀ and reduced coenzyme Q₁₀ in a sample by high performance liquid chromatography (HPLC) using an UV detector and calculating the ratio from the volume ratio obtained, or a method comprising using an HPLC system incorporated with an electrochemical detector for calculating the ratio of oxidized coenzyme Q₁₀ and reduced coenzyme Q₁₀ from the peak area. The HPLC system incorporated with an electrochemical detector makes it possible to specifically determine oxidation-reduction substances and has high sensitivity, and thus is highly useful for determining trace amount of reduced coenzyme Q₁₀ in the living body or samples. In the present invention, all the reduced coenzyme Q₁₀ ratios are determined by the HPLC system incorporated with an electrochemical detector.

The content of coenzyme Q₁₀ in the composition of the invention is preferably 0.001 to 99% by weight, and more preferably 0.01 to 20% by weight.

The aged mammals cited in the invention refer to, as for human, individuals of 40 years old or older. The use to humans of 60 years old or older is particularly preferable. As for rats, individuals of 50 weeks old or older are referred to. And as for other animals, individuals equivalent to humans of 40 years old or older are referred to.

The composition of the invention can be used in pharmaceutical products, functional foods, food materials, or the like. The functional foods cited herein refer to products for maintaining or improving health by oral intake of products other than pharmaceutical products, such as oral supplements, foods for specified health uses, health foods, or dietary supplements.

In producing the composition of the invention, the content, dosage form, preservation method, and preservation form of reduced coenzyme Q₁₀ can be arbitrary determined according to the application, such as use in pharmaceutical products, health foods or foods. The composition of the present invention is produced by mixing reduced coenzyme Q₁₀ with a carrier acceptable as a pharmaceutical preparation or food and, according to need, oxidized coenzyme Q₁₀ and/or an antioxidant. The carrier acceptable as a pharmaceutical preparation or food is not particularly restricted and there may be mentioned, for example, an excipient, a disintegrant, a lubricant, a binder, a colorant, a coagulation inhibitor, an absorption promoter, a solubilizing agent, a stabilizer, and the like.

The dosage form of the composition of the invention is not particularly restricted and may be, for example, solution preparations, powders, granules producible by adding a binder, powders coated with a coating agent, and capsule preparations producible by filling powders, granules or coated powders into capsules. The solution preparations are not particularly restricted and there may be mentioned, for example, a drink, an injection, an infusion, and the like. Also employable are soft capsule preparations producible by adding natural oil, higher fatty acid oil, a higher fatty acid monoglyceride, a surfactant, or mixture thereof, etc. and filling that in its oily state into capsules. In this case, as the capsule, gelatin-based ones or ones based on other soluble polymer substances than gelatin can be used, for example. Also, microcapsules are included in such capsules.

To the composition of the invention, it is desirable to add an antioxidant for protecting reduced coenzyme Q₁₀ in preparations from oxidation. In some dosage forms, however, the addition is not necessary. As the antioxidant usable in the invention, there may be mentioned citric acid, citric acid derivatives, vitamin C, vitamin C derivatives, lycopene, vitamin A, carotenoids, vitamin B, vitamin B derivatives, flavonoids, polyphenols, glutathione, pyrroloquinoline quinone, pycnogenol, flavangenol, selenium, sodium thiosulfate, vitamin E, vitamin E derivatives, superoxide dismutase (SOD), glutathione peroxidase, glutathione-S-transferase, glutathione reductase, catalase, ascorbic acid peroxidase, and mixtures of these.

The composition of the invention is capable of enhancing the antioxidative activity in blood by being administrated to aged mammals and therefore can be used for preventing or treating diseases related to oxidative stress. The diseases related to oxidative stress are not particularly restricted and there may be mentioned, for example, mitochondrial disease, Alzheimer's disease, Parkinson's disease, ischemic heart disease (myocardial infarction, angina pectoris, etc.), cardiac hypertrophy, heart failure, ischemia-reperfusion injury, arteriosclerosis, gastric mucosal disorder, inflammatory bowel disease, acute pancreatitis, nephrosclerosis, renal failure, nephritis, inflammatory skin disease, diabetes mellitus, diabetic complication, lung disease, retinal disease, cancer, cerebral apoplexy (cerebral infarction, intracranial hemorrhage, transient ischemic attack, etc.), and the like. Furthermore, diseases related to intravascular oxidative stress are not particularly restricted and there may be mentioned, for example, arteriosclerosis, ischemic heart disease (myocardial infarction, angina pectoris, etc.), cerebral apoplexy (cerebral infarction, intracranial hemorrhage, transient ischemic attack, etc.), nephrosclerosis, and the like. The term "administration" as used herein includes enteral and nonenteral administration, but enteral administration, particularly oral administration is preferred. The dose of reduced coenzyme Q₁₀ is not particularly restricted but, for humans, 10 to 300 mg/day per adult is preferred.

### (EFFECT OF THE INVENTION)

According to the present invention, a composition capable of keeping the reduced coenzyme Q₁₀ ratio in blood high, even in aged mammals, by coenzyme Q₁₀ intake, and therefore capable of maintaining antioxidative activity in blood high is provided.

### BEST MODE FOR CARRYING OUT THE INVENTION

The following Examples and Preparation Examples illustrate the present invention in further detail. These Examples and Preparation Examples are, however, by no means limitative of the scope of the invention.

In these Examples, the ratio of reduced coenzyme Q₁₀ to the total coenzyme Q₁₀ was determined using the HPLC system incorporated with an electrochemical detector (product of Shiseido Co., Ltd.) and a reduction column (product of Shiseido Co., Ltd.).

### (Reference Example 1)

10 SD rats (5-week-old, males) were divided into 2 groups each consisted of 5 rats so that each group had the same average body weight value. To one group, a solution of reduced coenzyme Q₁₀ in soybean oil (containing about 1% of oxidized coenzyme Q₁₀) was orally administered, and to the other group, a solution of oxidized coenzyme Q₁₀ in soybean oil was orally administered, each at the dose of 100 mg/kg body weight in coenzyme Q₁₀ equivalent. Then, the reduced coenzyme Q₁₀ ratio in plasma was determined with time lapses. Blood was collected after 1, 2, 4 and 8 hours of oral administration, and centrifuged to obtain plasma. The concentration of reduced coenzyme Q₁₀ and oxidized coenzyme Q₁₀ in the obtained plasma was quantified using HPLC in order to determine the reduced coenzyme Q₁₀ ratio.

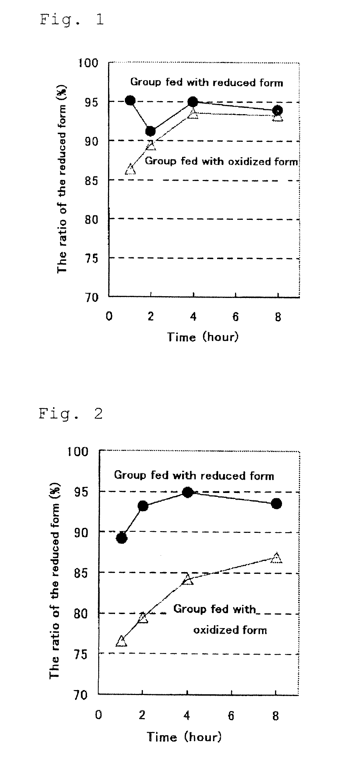

Fig.1 shows the change of the ratio of the reduced form of coenzyme Q₁₀ in plasma. It shows that there was almost no difference in the reduced coenzyme Q₁₀ ratio after 2 hours or later of administration between the administration of reduced coenzyme Q₁₀ and that of oxidized coenzyme Q₁₀.

### (Example 1)

10 SD rats (54-week-old, males) were divided into 2 groups each consisted of 5 rats so that each group had the same average body weight value. To one group, a solution of reduced coenzyme Q₁₀ in soybean oil (containing about 1% of oxidized coenzyme Q₁₀) was orally administered, and to the other group, a solution of oxidized coenzyme Q₁₀ in soybean oil was orally administered, each at the dose of 100 mg/kg body weight in coenzyme Q₁₀ equivalent. Then, the reduced coenzyme Q₁₀ ratio in plasma was determined with time lapses. Blood was collected after 1, 2, 4 and 8 hours of oral administration, and centrifuged to obtain plasma. The concentration of reduced coenzyme Q₁₀ and oxidized coenzyme Q₁₀ in the obtained plasma was quantified using HPLC in order to determine the ratio of the reduced form.

Fig.2 shows the change of the ratio of the reduced form of coenzyme Q₁₀ in plasma. It shows that, when reduced coenzyme Q₁₀ was administered, the ratio of the reduced form remained around 90%. Comparatively, when oxidized coenzyme Q₁₀ was administered, the ratio of the reduced form in plasma was about 76% after 1 hour of administration. The ratio had a tendency to increase with the lapse of time but, even after 8 hours of administration, the ratio remained about 87%, and did not increase to 90%.

### (Example 2)

10 SD rats (62-week-old, males) were divided into 2 groups each consisted of 5 rats so that each group had the same average body weight value. To one group, a solution of reduced coenzyme Q₁₀ in soybean oil (containing about 1% of oxidized coenzyme Q₁₀) was orally administered, and to the other group, a solution of oxidized coenzyme Q₁₀ in soybean oil was orally administered, each at the dose of 100 mg/kg body weight in coenzyme Q₁₀ equivalent. Then, the reduced coenzyme Q₁₀ ratio in plasma was determined with time lapses. Blood was collected after 1, 2, 4 and 8 hours of oral administration, and centrifuged to obtain plasma. The concentration of reduced coenzyme Q₁₀ and oxidized coenzyme Q₁₀ in the obtained plasma was quantified using HPLC in order to determine the ratio of the reduced form.

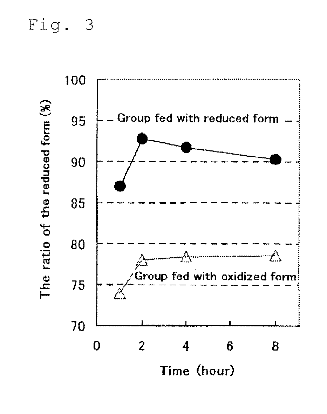

Fig.3 shows the change of the ratio of the reduced form of coenzyme Q₁₀ in plasma. It shows that, when reduced coenzyme Q₁₀ was administered, the ratio of the reduced form remained around 90%. Comparatively, when oxidized coenzyme Q₁₀ was administered, the ratio of the reduced form in plasma was about 74% after 1 hour of administration. The ratio had a tendency to slightly increase with the lapse of time but, even after 8 hours of administration, the ratio remained about 78%.

### (Preparation Example 1)

Olive oil was warmed to 60°C, and reduced coenzyme Q₁₀ (containing about 1% of oxidized coenzyme Q₁₀) melted at 60°C was added for dissolution. Thereto, vitamin E was added little by little for attaining a uniform mixture, and soft capsules were prepared in the conventional manner. Soft capsule preparations containing 20 mg of reduced coenzyme Q₁₀ in each capsule were obtained.

| | |
|---|---|
| Reduced coenzyme Q₁₀ | 20 parts by weight |
| (containing about 1% of oxidized coenzyme Q₁₀) | |
| Vitamin E | 15 parts by weight |
| Olive oil | 350 parts by weight |

### (Preparation Example 2)

Reduced coenzyme Q₁₀ (containing about 1% of oxidized coenzyme Q₁₀) was dissolved in acetone, and then allowed to be adsorbed on crystalline cellulose (fine powders) in order to be dried. The obtained product was mixed with corn starch to give powder preparations in the conventional manner.

| | |
|---|---|
| Reduced coenzyme Q₁₀ | 10 parts by weight |
| (containing about 1% of oxidized coenzyme Q₁₀) | |
| Crystalline cellulose (fine powders) | 40 parts by weight |
| Corn starch | 55 parts by weight |

### (Preparation Example 3)

Reduced coenzyme Q₁₀ (containing about 1% of oxidized coenzyme Q₁₀) was dissolved in acetone, and then allowed to be adsorbed on crystalline cellulose (fine powders) in order to be dried. The obtained product was mixed with corn starch, lactose, carboxymethyl cellulose, and magnesium stearate. Then, an aqueous solution of polyvinylpyrrolidone was added thereto as a binder and the mixture was granulated in the conventional manner to obtain powders. With these powders, talc was mixed as a lubricant, and tablets containing 20 mg of reduced coenzyme Q₁₀ in each tablet were obtained.

| | |
|---|---|
| Reduced coenzyme Q₁₀ | 20 parts by weight |
| (containing about 1% of oxidized coenzyme Q₁₀) | |
| Corn starch | 25 parts by weight |
| Lactose | 15 parts by weight |
| Calcium carboxymethyl cellulose | 10 parts by weight |
| Crystalline cellulose (fine powders) | 40 parts by weight |
| Polyvinylpyrrolidone | 5 parts by weight |
| Magnesium stearate | 3 parts by weight |
| Talc | 10 parts by weight |

### (Preparation Example 4)

The following ingredients were granulated in the conventional manner and filled into gelatin hard capsules. Capsule preparations containing 20 mg of reduced coenzyme Q₁₀ in each capsule were obtained.

| | |
|---|---|
| Reduced coenzyme Q₁₀ | 20 parts by weight |
| (containing about 1% of oxidized coenzyme Q₁₀) | |
| Crystalline cellulose (fine powders) | 40 parts by weight |
| Corn starch | 20 parts by weight |
| Lactose | 62 parts by weight |
| Magnesium stearate | 2 parts by weight |
| Polyvinylpyrrolidone | 3 parts by weight |

### (Preparation Example 5)

Rapeseed oil was warmed to 60°C, and reduced coenzyme Q₁₀ (containing about 1% of oxidized coenzyme Q₁₀) melted at 60°C was added for dissolution. Thereto, beeswax was added as a stabilizer and soft capsules were prepared in the conventional manner. Soft capsule preparations containing 50 mg of reduced coenzyme Q₁₀ in each capsule were obtained.

| | |
|---|---|
| Reduced coenzyme Q₁₀ | 50 parts by weight |
| (containing about 1% of oxidized coenzyme Q₁₀) | |
| Rapeseed oil | 300 parts by weight |
| Beeswax | 40 parts by weight |

### (Comparative Preparation Example 1)

Soft capsule preparations containing oxidized coenzyme Q₁₀ were obtained in the same manner as Preparation Example 5 except that oxidized coenzyme Q₁₀ was used in lieu of reduced coenzyme Q₁₀.

| | |
|---|---|
| Oxidized coenzyme Q₁₀ | 50 parts by weight |
| Rapeseed oil | 300 parts by weight |
| Beeswax | 40 parts by weight |

### (Example 3)

Two soft capsules obtained in Preparation Example 5 and Comparative Preparation Example 1 (containing 100 mg of coenzyme Q₁₀) were fed to two groups of healthy aged people (20 male and female individuals in each group, age: 64 to 77 years old), and the effect on the reduced coenzyme Q₁₀ ratio in plasma was evaluated. The results are shown in Table 1.

**Table 1**

| | Change of reduced coenzyme Q₁₀ ratio (%) in plasma | |
|---|---|---|
| | After 2 hours of feeding | After 6 hours of feeding |
| Group fed with oxidized coenzyme Q₁₀ | -0.86 | -0.09 |
| Group fed with reduced coenzyme Q₁₀ | 0.05 | 0.51 |

In the group fed with oxidized coenzyme Q₁₀, the ratio of reduced coenzyme Q₁₀ to the total coenzyme Q₁₀ in plasma rather decreased after 2 hours of feeding, and the ratio recovered to almost the same level as before feeding after 6 hours of feeding. This result is nearly identical to the result shown in Non-Patent Document 5. It is confirmed that feeding of oxidized coenzyme Q₁₀ rather decreases the ratio of the reduced form in plasma. It is supposedly resulted from lowered antioxidative activity due to consumption of the reducing equivalent in bodies for reducing the oxidized form. On the contrary, in the group fed with reduced coenzyme Q₁₀, the ratio of reduced coenzyme Q₁₀ to the total coenzyme Q₁₀ in plasma increased with the lapse of time since 2 hours after administration, which shows the antioxidative activity in bodies increased.

### BRIEF DESCRIPTION OF THE DRAWINGS

Fig. 1 is a line graph showing the time course of reduced coenzyme Q₁₀ ratio in plasma of 5-week-old SD rats according to Reference Example 1. The ordinate shows the ratio (%) of the reduced form to the total coenzyme Q₁₀ in plasma. The abscissa shows the lapse of time after administration of coenzyme Q₁₀. The symbols shows as follows; •: the group fed with reduced coenzyme, and □: the group fed with oxidized coenzyme Q₁₀. The values plotted on the graph are the average value of 5 rats in each group.
Fig. 2 is a line graph showing the time course of reduced coenzyme Q₁₀ ratio in plasma of 54-week-old SD rats according to Example 1. The ordinate shows the ratio (%) of the reduced form to the total coenzyme Q₁₀ in plasma. The abscissa shows the lapse of time after administration of coenzyme Q₁₀. The symbols shows as follows; •: the group fed with reduced coenzyme Q₁₀, and □: the group fed with oxidized coenzyme Q₁₀. The values plotted on the graph are the average value of 5 rats in each group.
Fig. 3 is a line graph showing the time course of reduced coenzyme Q₁₀ ratio in plasma of 62-week-old SD rats according to Example 2. The ordinate shows the ratio (%) of the reduced form to the total coenzyme Q₁₀ in plasma. The abscissa shows the lapse of time after administration of coenzyme Q₁₀. The symbols shows as follows; •: the group fed with reduced coenzyme Q₁₀, and □: the group fed with oxidized coenzyme Q₁₀. The values plotted on the graph are the average value of 5 rats in each group.

## Claims

1. A composition
which comprises reduced coenzyme Q₁₀ represented by the following formula (1) as an active component.

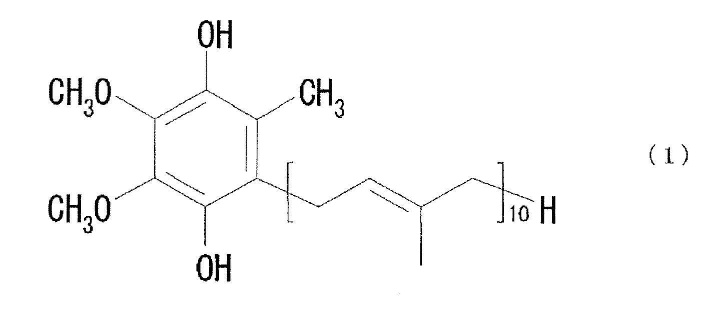

2. The composition according to Claim 1,
which is administered to aged mammals.

3. The composition according to Claim 1 or 2,
which enhances the antioxidative activity in blood of aged mammals.

4. The composition according to any one of Claims 1 to 3,
which further comprises oxidized coenzyme Q₁₀ represented by the following formula (2) as an active component.

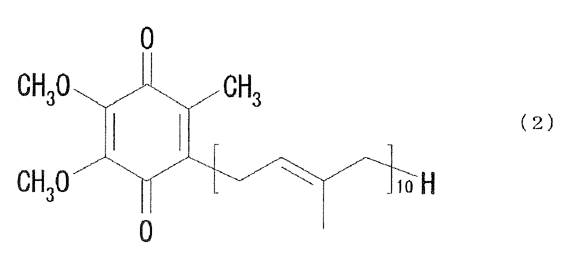

5. The composition according to any one of Claims 1 to 4,
which further comprises an antioxidant.

6. The composition according to any one of Claims 1 to 5,
which is used for treating or preventing diseases related to oxidative stress.

7. The composition according to any one of Claims 1 to 5,
which is used for treating or preventing diseases related to intravascular oxidative stress.

8. A method for producing a composition
which comprises mixing reduced coenzyme Q₁₀ with a carrier acceptable as a pharmaceutical preparation or food.

9. The method according to Claim 8,
wherein the composition is administered to aged mammals.

10. The method according Claim 8 or 9,
wherein the composition enhances the antioxidative activity in blood of aged mammals.

11. A method for enhancing the antioxidative activity in blood
which comprises administering reduced coenzyme Q₁₀.

12. The method according to Claim 11,
which comprises administration to aged mammals.
